Europäisches Patentamt

European Patent Office

Office européen des brevets

(11) Publication number: **0 262 972 A2**

## EUROPEAN PATENT APPLICATION

(21) Application number: 87308720.9

(22) Date of filing: 01.10.87

(51) Int. Cl.⁴: **A 01 H 1/00**
C 12 N 15/00, C 12 N 5/00,
A 01 G 7/00

(30) Priority: 01.10.86 US 913914

(43) Date of publication of application:
06.04.88 Bulletin 88/14

(84) Designated Contracting States:
AT BE CH DE ES FR GB GR IT LI LU NL SE

(71) Applicant: THE PLANT CELL RESEARCH INSTITUTE INC.
6560 Trinity Court
Dublin California 94568 (US)

(72) Inventor: Trulson, Anna J.
7000 Tesla Road
Livermore California 94550 (US)

Shaheen, Elias A.
2920 Trotter Way
Walnut Creek California 94596 (US)

(74) Representative: Goldin, Douglas Michael et al
J.A. KEMP & CO. 14, South Square Gray's Inn
London WC1R 5EU (GB)

(54) Genetic transformation and controlled regeneration of cucumis SP in vitro.

(57) Transformation of Cucumis sp. plants with A. rhizogenes followed by regeneration into gentically altered plants. Inverted hypocotyls of C. sativus L., cv, Straight Eight were inoculated with A. rhizogenes containing the vector pARC8 or pARC16 containing NOS/NPT chimeric marker gene conferring resistance to kanamycin in addition to the resident Ri plasmid. Roots produced as the inoculated hypocotyls were excised and sequentially regenerated in CTM-2, -3 and -4 media, resulting in mature, fruiting transgenic plants having germinable seed. Short intermodal morphology was expressed yielding Cucumber plants, called "PCRI Hi Density" , having greater yield per acre.

Fig. I.

## Description

"GENETIC TRANSFORMATION AND CONTROLLED REGENERATION OF CUCUMIS SP IN VITRO"

Cross Reference to Related applications:

The genetic transformation methods of this invention may be followed by explant regeneration techniques set forth in our copending explant regeneration case (our reference N 43003, hereinafter referred to as case 92/9) filed of even date herewith, or by protoplast isolation and regeneration techniques set forth in our copending protoplast regeneration case (our reference N 43004, hereinafter referred to as case 92/13) filed of even date herewith. Likewise, the somaclonal variants produced by the techniques of case 92/9 or somatic hybrids produced by protoplast/cytoplast/sphereoplsast/liposome fusion (92/13) may be preceded by and combined with the transformation techniques of this case. The disclosures of those cases are hereby incorporated by reference herein to the extent needed.

Field:

This invention relates to genetic transformation of Cucumis sp. plants by use of Agrobacterium rhizogenes and subsequent regeneration in vitro into genetically altered plants. More specifically, this invention relates to transformation of Cucumis sp. plants by inoculation of inverted hypocotyl sections with A. rhizogenes, induction of roots thereon, and regeneration of genetically altered plants from the root tissue. The regeneration can be achieved directly from the induced roots, or from explants of tissue from the roots, stem, or leaves of plantlets or mature plants, employing CTM-1 to -3 culture media, or from protoplasts isolated from such tissue employing CMP-1 to -3 media. The transformation techniques of this invention, when combined with regeneration, somatic hybridization, and somaclonal variation screening, permit a complete range of techniques for genetic improvement in plants, and more specifically, in the genus Cucumis. Transgenic plants of this invention called "PCRI HiDensity" are characterized as having a compact morphology (short internodes).

Background

Sterility barriers between species are among the most limiting factors in plant breeding. They preclude transfer of many desirable traits such as disease, insect and herbicide resistance between species of cultivated or week plants because of sexual incompatibility. This problem is especially acute in the family Cucurbitaceae, which includes the genera Cucumis (cucumbers [C. sativus], and melons [C. melo]), Citrullus (watermelons) and Cucurbita (squash.) Among the cultivated species of the genus Cucumis, successful sexual crosses can be made only between C. sativus and the closely related C. harwickii. Attempts to interbreed cucumbers and other species in this genus failed (Deakin et al., 1971).

The recent advances in genetic engineering seem to be a promising alternative to sexual propagation techniques for improvement of this economically important crop. This is particularly true in the case of tissue culturing where an increase in genetic variation, so-called somaclonal variation, is noted (Larkin and Scowcroft 1981). Further, sterility barriers may be overcome by fusion of protoplasts of sexually incompatible species. However, the lack of a reliable method for regeneration of Cucumis plants by explant tissue culture techniques has prevented significant progress until our recent developments of both: a) the CTM-1 through CTM-4 series of explant embryoid generation and development media, as reported by us in Trulson, A.J. and Shahin, E.A., "In vitro plant regeneration in the genus Cucumis" (in press 1986) and covered in our copending application (92/9); and b) the CPM-1 through CPM-3 series of protoplast regeneration and development media, also reported by us in the aforesaid paper (Trulson and Shahin, in press 1986) and covered in our copending application (92/13).

Still another route to overcoming sterility barriers is the introduction of desirable traits by gene transfer and subsequent expression thereof. Inter- and intraspecific gene transfer is a major objective of genetic engineering. Successful expression of transferred genes in transformed plants is possible and has already been achieved in several systems (Fraley et al., 1983; Herrera-Estrella et al., 1983; Bevan 1984; Horsch et al., 1985; Jones et al., 1985). This success is the basis for the great hopes for future crop improvement through the incorporation of various desirable traits. The application of genetic engineering would be especially welcome in the improvement of the genus Cucumis, particularly cucumbers, since sexual incompatibility between cucumbers and other members of the family Cucurbitaceae precludes transfer of such traits as disease and insect resistance using convention breeding methodology (Deakin et al., 1971).

The most widely used method of gene transfer is via a disarmed form of the Ti plasmid of the soil bacteria Agrobacterium tumefaciens (Zambryski et al., 1983). A. tumefaciens is a plant pathogen that causes crown-gall tumors after infection of wounded dicotyledonous plants. Large plasmids (Ti-plasmids) are responsible for the oncogenicity of the bacterium. Crown-gall tumours contain a DNA segment, called the T-DNA, which is homologous with a defined part of the Ti-plasmid present in the tumor-inducing bacterium, and is stably integrated into the plant genome. Apart from the T-DNA, another region of the Ti-plasmid-called the vir-region, is essential for tumour induction (Hoekema et al., 1983). An alternative tool for gene transfer is A. rhizogenes, which differs from A. tumefaciens by inducing roots rather than tumours (Chilten et al. 1982; David et al., 1984).

Binary Ti plasmid vector systems consist of two plasmids in Agrobacterium, where one plasmid contains the DNA that can be transferred to plant cells and the other contains the virulence (vir) genes which are necessary for the DNA transfer but are not themselves transferred. Hoekema et al. (1983) reported the interaction in A. tumefaciens of two compatible plasmids, one containing the vir-region, the other carrying the T-DNA on a wide host-range replicon. An A. tumefaciens strain harboring both plasmids has a normal tumor-inducing capacity, although neither plasmid is functional alone. With this approach, the T-DNA on one plasmid can, becaue of it size, be easily genetically manipulated using Escherichia coli as a host. Transfer of this plasmid into an A. tumefaciens strain harboring the plasmid with the vir-region allows introduction of the manipulated T-DNA into plant cells. In this way, sophisticated binary vector systems for plant genetic engineering can be developed.

Simpson et al. (in press 1986) constructed two non-oncogenic vectors (pARC4 and pARC8) based on the binary Ti plasmid system of Agrobacterium tumefaciens for plant transformation. Each vector contains the left and right termini sequences from pTiT37. These sequences, which determine the extent of DNA transferred to plant cells, flank unique restriction enzyme sites and a marker gene that functions in the plant (nopaline synthase in pARC4, or neomycin phosphotransferase in pARC8). After construction in vitro, the vectors can be conjugatively transferred from E. coli to any of several Agrobacterium strains containing vir genes.

Using A. rhizogenes strain A4 containing the resident Ri plasmid plus a vector with the nopaline synthase marker, Simpson et al (in press, 1986) found that up to 50% of the hairy roots resulting from the infection of alfalfa or tomato synthesized nopaline Vector DNA encoding a screenable marker was frequently co-transferred with Ri plasmid DNA to an alfalfa or a tomato cell. In contrast, they found the frequency of co-transfer to soybean cells difficult to estimate because they encountered a high background of non-transformed roots using that species. Up to five copies of the vector DNA between the terminal sequences were faithfully transferred and maintained in most cases suggesting that the termini sequences and the vir genes from the Ri and Ti plasmids are functionally equivalent. Simpson et al (in press 1986) did not study the family Cucurbitaceae or the genus Cucumis.

To our knowledge no one has been able to successfully transfer genetic material into Cucumis sp. plant tissue via either A. tumefaciens or A. rhizogenes with regeneration into a genetically altered plant structure (cell, cell colony, tissue, mini-calli, embryoid, plantlet, or plant) which expressed the transferred gene. Thus, to date, this avenue of genetic engineering for Cucumis sp. has remained closed.

Accordingly, there is a need for an efficient, facile and reproducible method by which A. rhizogenes can be used successfully to transfer a foreign gene into Cucumis sp. plant structures or plants, which methodology opens new avenues in genetic improvement of Cucumis sp. by permitting the transfer of agronomically desirable genes. Additionally, there is a need for a technique for introduction of marker genes, such as kanamycin resistance, to facilitate somatic inter- and intraspecies hybridization/cybridization of Cucumis sp. with other species.

## THE INVENTION

### Objects

It is among the objects of the invention to provide methods for the genetic transformation of plant structures and/or plants by use of Agrobacterium rhizogenes, and more particularly, transformation of Cucumis sp. plant tissue.

It is another object of the invention to provide methods of in vitro regeneration of transformed Cucumis sp. plant tissue into competent, genetically altered mini-calli, embryoids, roots, plantlets and plants.

It is another object of this invention to provide processes for the in vitro generation, induction and regeneration of cells, cell colonies, mini-calli, embryoids and plantlets from genetically altered protoplasts or explant tissue which are capable of developing into mature plants.

It is another object of this invention to provide methods of continuously producing genetically altered plant mini-calli and embryoids for encapsulation as artificial seeds.

It is another object of this invention to apply the novel methods and media of this invention to the production of improved plants in the genus Cucumis.

It is another object of this invention to shorten the time for producing genetically engineered and/or hybridized plant stock without having to go through full seed development.

It is another object of this invention to asexually propagate plants without hormonal or chemical induction of flowers.

It is another object of this invention to provide methods for introducing foreign or selectable marker genes into Cucumis sp., inter alia, to facilitate somatic hybridization.

It is another object of the invention to produce genetically altered mini-calli and embryoids which may be stored frozen, followed by later thawing and continuation of maturation and development into genetically altered plantlets and/or plants.

It is another object of this invention to provide methods of transforming Cucumis sp. tissue to facilitate asexual somatic hybridization.

It is another object of this invention to introduce one or more vector T-DNA(s) alone, the Ri-plasmid T-DNA alone, or both vector and Ri plasmid T-DNA(s), of A. rhizogenes into Cucumis sp. plants with expression of one or the other, or both.

It is another object to introduce resistance to kanamycin, chloramphenicol, or other antibiotics, to Cucumis sp. plants as marker genes for genetic engineering and trait selection.

Still other objects of this invention will be evident from the balance of this specification and claims.

Definitions

The terminology used herein is not intended to vary from the terminology used in the field. However, the meaning of some terms used in the field is not necessarily uniform, and the following definitions will be of help in this case:

"Transformation refers to genetic alteration of a plant by introduction, and stable and heritable incorporation, into the subject plant DNA of a foreign (plant, bacterial, viral or chimeric) DNA.

"Plantlet refers to a plant sufficiently developed to have a shoot and a root that is asexually reproduced by cell culture.

"Explant refers to a section or a piece of tissue from any part of a plant for culturing.

"Hormone" refers to a plant growth regulator that affects the growth or differentiation of plants, and is exogenous as used in reference to the various media herein.

"Callus", and its plural "calli" refer to an unorganized group of cells formed in response to a cut, severing or injury of a plant, and herein refers to the unorganized cell growth which may form on explant tissues during culturing or from division of protoplasts which have regenerated cell walls.

"Embryoid refers to a structure similar in appearance to plant zygotic embryo.

"Somatic Hydrid" and "Somatic Hybridization" refers generally to stable combination of cellular material, be it protoplast/protoplast or protoplast/cytoplast combinations, and includes cybrids and cybridization.

Abbreviations

NAA = alpha-naphthaleneacetic acid

ZR = Zeatin Riboside

BAP = 6-benzylaminopurine

GA$_3$ = Giberellic acid

2,4-D = 2,4-dichlorophenoxyacetic acid MS Medium - Murashige and Skoog Medium (Murashige and Skoog, 1962)

CPM = Cucumis Protoplast Medium, as in CPM-1, -2, -3 (Trulson and Shahin, 1986)

CPE = Cucumis Protoplast Enzyme solution

CPE-G = Cucumis Protoplast Enzyme solution containing glycine

CTM = Cucumis Transformation Medium, as in CTM-1, -2, -3, -4 (Trulson and Shahin, 1986)

PET Solution = Tomato pre-enzymatic solution (Shahin, 1985)

TM-1 = Tomato Medium 1 (Shahin, 1985)

TM-2 = Tomato Medium 2 (Shahin, 1985)

pARC8 = A binary vector introduced in A. rhizogenes (Simpson et al., 1986)

pARC16 = A modified pARC8 binary vector having a 9.0 kb Hind III fragment at the Hind III site of the T-DNA

Hind III = A well-known restriction enzyme

AB Medium = Agrobacterium Medium (Chilton et al., 1974)

Drawings

The disclosures herein have reference to the drawings in which:

Figure 1 is a schematic of the pARC8 vector plasmid used in the transformation of this invention;

Figure 2 is a reproduction of the NPT II test to select for kanamycin resistance (+) or absence of resistance (-);

Figure 3 is a reproduction of the Southern blot Hind III fragment analysis demonstrating integration of the Vector DNA into the plant genome; and

Figure 4 is a photographic reproduction of a transgenic plant of this invention compared to a normal control.

Summary

Transgenic cucumber plants were regenerated from roots induced by inoculation of inverted hypocotyl sections of Cucumis sativus L., cv. Straight Eight with Agrobacterium rhizogenes containing the vector pARC8 or pARC16 in addition to the resident Ri-plasmid.

Roots produced on the surfaces of inverted hypocotyls inoculated with A. rhizogenes were excised, and a first series were placed on CTM-2 medium (comprising MS salts, 5μM 2,4-D 5 μ M NAA and 2 μM BAP in 0.7% agar), followed by culturing for 2 - 3 weeks under continuous light (3500 lux), at 27°C. A second series of inoculated roots were cultured on CTM-2 medium supplemented with 25 mg/l of kanamycin (Sigma). Embryoids that appeared on the root surface were detached and transferred to CTM-3 Medium (MS with 5μM NAA and 2μM BAP in 0.7% agar), and cultured in the same conditions for 10 - 14 days. Mature embryoids were transferred onto hormone-free MS medium (1% agar) on which shoots were produced. To eliminate bacterial carry-over, media contained 100 mg/l of cefotaxime.

The DNA transferred to the plant from the vector (T-DNA) included a gene which encoded the enzyme neomycine phosphotransferase II, and thus conferred on the plant cells resistance to kanamycin. The transgenic plants looked normal and were positive for the neomycin phosphotransferase II. Southern blot analysis of the transgenic plants revealed that all plants contained vector DNA, but only some of them contained DNA from the Ri plasmid.

The transgenic plants of this invention produced fruit having germinable seed which has been carried into the R-2 generation, and is called "PCRI HiDensity", plants of which are characterized as having a compact morphology (short internodes) permitting closer plant spacing, with attendant greater production per acre.

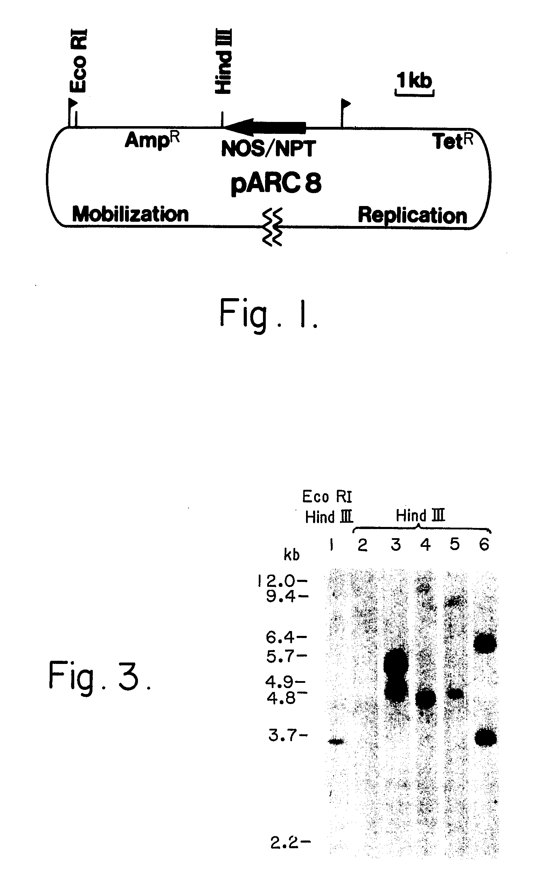

Figure 1 shows a schematic of the disarmed pARC8 vector plasmid used in the transformations of this invention. The vector contains a wide host range replicon (to permit the replication of the plasmid in both Escherichia coli and Agrobacterium), a bacterial origin of transfer (which permits the vector to be mobilized by a helper plasmid), and markers encoding resistance to tetracycline (TETR) and ampicillin (AmpR), which allow selection of bacteria containing the vector. The unique restriction enzyme sites Eco Ri and Hind III facilitate the insertion in vitro of "foreign DNA fragments into the vector. The flags mark the termini sequences which Agrobacterium uses to delimit the DNA it transfers to plants cells. The NOS/NPT is a selectable marker which confers resistance to kanamycin in transformed plant cells. This chimeric gene consists of the neomycin phosphotransferase (NPT) coding region, flanked by the nopaline synthase (NOS) promoter and terminator. The pARC16 is a modified pARC8 vector.

Detailed Description of the Best Mode of Carrying Out the Invention

The following detailed description is by way of example and not by way of limitation of the principles of this invention, and has reference to specific examples in which transformation by A. rhizogenes followed by regeneration, embryoid generation from induced roots and plantlet development therefrom, within the scope of this invention is described for cucumber (Cucumis sativus L.) by way of example.

EXAMPLE A. Transformation of Cucumis sp.

1. Media Composition

The composition of the various media employed herein are as set forth in the corresponding references given above, unless otherwise noted herein.

2. Source of Cucumis Hypoctyl Sections for Inoculation.

Seeds of cucumber cv. Straight Eight (ARCO Seed, Brooks, Oregon) are sterilized in 10% (v/v) Clorox (commercial bleach containing 5.25% sodium hypochlorite) with a drop of Tween 80 (one droplet per 100 ml of the sterilizing solution) for 10 minutes, then rinse 3x in sterile, distilled water and placed in sterile petri plates (ca 30 per plate) lined with moist Whatman #3 filter paper. To assure uniform and rapid germination of the seeds, plates are kept for 24 - 30 hours at 27°C, in the dark. Germinated seeds (radicle length ca 5mm) are placed aseptically in Magenta boxes (six seeds per box) containing ca 40 ml of TM-1 medium (Shahin, 1985) supplemented with 150 mg/l of Carbenicillin (Sigma), and incubated for four days in a growth chamber, at 21°C night, 26°C day, 14 hour photoperiod (4500 lux). When the seedlings are green, but the cotyledons only partially unfolded, Magenta boxes are placed for 14 hours in the dark, at room temperature.

3. Vector plasmid and Agrobacterium strain.

We used the A4 strain of A. rhizogenes containing, in addition to the resident Ri-plasmid, a vector pARC8 (see Figure 1) derived from the Ti plasmid of A. tumefaciens (Simpson et al., in press 1986), or a vector pARC16, which is a modified pARC8 vector in which a 9.0kb Hind III restriction fragment inserted at the Hind III site of the T-DNA region. The methods and binary vector of this system is disclosed and claimed in copending application Serial Number 634,283, filed July 25, 1984, the disclosure of which is hereby incorporated by reference herein to the extent required. As shown in Figure 1, the selectable marker in pARC8 (and in pARC16), which conferred resistance to kanamycin, is NOS/NPT, a chimeric gene constructed from Tn5 neomycin phosphotransferase (NPT) coding region flanked by the nopaline synthase (NOS) promoter and terminator.

4. Inoculation Procedure.

The inoculum consisted of four-day-old culture of the above A-4 (pARC8 or pARC16) A. rhizogenes strain grown at room temperature in the dark, on AB medium (Chilton et al., 1974) supplemented with 5 mg/l of tetracycline (Sigma) to select for the bacterial TetR marker on the vector. The inoculum was collected on sterile bacteriological loop and smeared gently on the cut surface of inverted hypocotyl sections (approx. 2 cm long) of the seedlings of 1 above which were placed in hormone-free MS medium (Murashige and Skoog, 1962). The plates were then sealed and incubated at 27°C, under continuous light (2500 lux). One week after inoculation, the hypocotyl sections were cut above the agar surface and transferred into hormone-free MS medium supplemented with 100 mg/l of the antibiotic cefatoxime (Calbiochem), followed by incubation in the same conditions. Cefatoxime, an ampicillin analogue, was used since it is not modified by the beta-lactamase encoded by the AmpR gene on the vectors pARC8 or pARC16.

## 5. Plant regeneration using CTM Media. Test Series A and B.

In all media below, the pH was adjusted to 5.8 prior to autoclaving at 121°C for 15 minutes. To eliminate bacterial carry-over, the media contained 100mg/l of cefotaxime.

In a first series of tests (Series A), roots (5 to 10 mm in length) produced on the inoculated surfaces were excised and placed on CTM-2 Medium (comprising of MS salts, $5\mu$ M 2,4-D (Sigma), 5 $\mu$ M NAA (Sigma), 2 $\mu$ M BAP (Sigma) in 0.7% agar) followed by culturing fo 2 - 3 weeks under continuous light (3500 lux) , at 27°C.

In a second series (Series B), the roots were excised and cultured in CTM-2 Medium supplemented with 25 mg/1 of kanamycin (Sigma) to select tranformed plants.

In both series of tests, embryoids that appeared on the root surface after 2 - 3 weeks were detached and transferred to CTM-3 Medium (MS medium having 5 $\mu$M NAA and 2$\mu$M BAP in 0.7% agar) and cultured under the same conditions for 10 - 14 days to develop mature embryoids.

These mature embryoids were transferred onto CTM-4 medium (hormone free MS medium solidified with 1% agar) on which shoots (plantlets) were produced.

These regenerated plantlets were transplanted to a mixture (1:1,v/v) of peatlite (Jiffy Products Co., West Chicago IL.) and soil for hardening and development into plants.

## 6. Neomycin phosphotransferase (NPT) assay..

The frequency of transformation by vector DNA was assessed in the regenerated plants of both Series A and B using an assay for NPT (Reiss et al., 1984). Small pieces (approximately 25 mm$^2$) of leaf tissue were used in the assay. The assay was performed twice, initially on plantlets on the hormone-free CTM-4 medium, and later on plants, 2 - 4 weeks after they had been potted and hardened in the soil mixture.

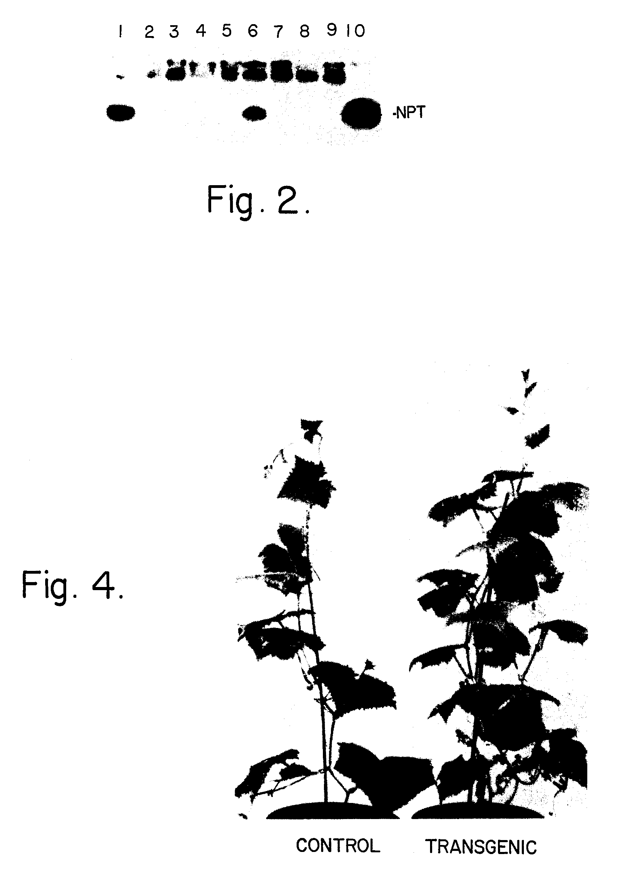

Figure 2 shows the neomycin phosphotransferase II test on cucumber plants regenerated from roots induced by inoculation with A. rhizogenes in accord with this invention. We used the native polyacrylamide gel assay of Reiss et al. (1984). Numbers 1 - 9 represent samples from randomly chosen cucumber plants regenerated without selection on kanamycin, sample No. 10 is a positive control (bacteria producing NPT). "NPT" indicates mobility of the enzyme.

## 7. Isolation of DNA and Southern blot analysis.

Southern blot analysis (Southern, 1975) was used to confirm the integration of the NOS/NPT gene in the DNA of the NPT-positive plants. The DNA was isolated from 2 grams (fresh weight) of young leaf tissue according to the procedure of Saghai-Maroof et al. (1984), then digested with Hind III and electrophoresed on an agarose gel, blotted and probed essentially as described by Thomashow et al. (1980). Plasmid pNEO 105 (Simpson et al., 1986; copending application Serial No. 634,283) containing the chimeric gene NOS/NPT, was used as the probe of the transferred portion of the vector DNA. Southern blot analysis was also used to investigate the extent of the Ri-plasmid DNA transfer into the DNA of the NPT-positive plants. Plasmids pFW94 and pFW41 (Huffman et al., 1984), which are clones of the Ri plasmid T-DNA, were used as probes to determine the presence of the $T_L$-DNA and $T_R$-DNA, respectively.

Figure 3 shows the southern blot hybridization analysis of cucumber plant DNA demonstrating integration of the vector DNA into the cucumber genome. Lane 1: half-copy reconstruction of the chimeric NOS/NPT gene containing Eco Ri- and Hind III- digested pNEO 105 which contains the NOS/NPT gene cloned in pBR322 (Simpson et al., 1986). Lane 2: Hind III-digested DNA from non-transformed, control cucumber plant. Lanes 3 - 6: Hind III-digested DNA from NPT-positive plants regenerated from transformed roots. The probe is pNE0105.

## 9. Results and Discussion

The A4 strain of A. rhizogenes containing vectors pARC8 or pARC16 infected Cucumis sp. cells, as exemplified by the cucumber hypocotyls above, as indicated by dense, cream-colored callus that appeared on inoculated surfaces after 7 - 10 days. One or two weeks later this callus produced roots. Control, uninoculated hypocotyl fragments produced small amounts of white, loose callus but did not produce roots.

A total of 691 roots harvested from the inoculated hypocotyl sections were plated on the embryo-induced CTM-2 Medium. Of these, over 9.2% (64 roots) regenerated into plantlets. This is a similar ratio as was obtained with non-transformed root explants. Among each of the 64 plantlets regenerated from a separated root, 22 plantlets were positive in the test for the neomycin phosphotransferase II (NPT-positive). These plants remained NPT-positive when assayed a second time after they were potted in the soil mix. Table I below summarizes the results.

0 262 972

TABLE I

Transformation of cucumber (Cucumis sativus L., cv. Straight Eight) by A. rhizogenes with and without selection on kanamycin.

| Selection Agent | Number of roots cultured | Number of roots that regenerated into plantlets | Number of NPT-positive plantlets |
|---|---|---|---|
| - kanamycin | 126 | 11 | 2 |
| + kanamycin | 565 | 53 | 20 |
| Totals | 691 | 64 | 22 |

As noted above in Table I, without selection for resistance to kanamycin two out of eleven regenerated plants were NPT positive, whereas when kanamycin (25mg/l) was added to the embryo-inducing CTM-2 medium, some 40% of the regenerated plants were NPT-positive. Figure 2 shows the NPT test for 9 of the 11 plants, with Nos. 1 and 6 showing positive; No. 10 in Figure 2 is the positive control. The addition of 25 mg/l kanamycin did not affect the regeneration process of the transformed tissue, nor did it prevent regeneration of some NPT-negative plants. This concentration of kanamycin (25 mg/l) also allowed some growth of the control (non-tranformed) roots; however, the controls did not regenerate plants in the presence of kanamycin.

In another series of tests (Series C), kanamycin concentration was doubled to 50 mg/l, but this concentration of kanamycin slowed plant regeneration and increased the number of abnormal plantlets.

Southern blot analysis of the DNA from the NPT-positive plants (Figure 3) confirmed the integration of the vector T-DNA in cucumber DNA. Each transformed plant contained a single copy of foreign DNA, as indicated by the presence of two bands corresponding to two border fragments (Figure 1) resulting from the Hind III digest. DNA was isolated from five independent, NPT-positive plants, digested by Hind III and analyzed using Southern blots. The probes were pNEO 105 (vector DNA; Simpson et al., 1986), pFW94 or pFW41 (for $T_L$-DNA and $T_R$-DNA, respectively; Huffman et al., 1984). Among the five plants assayed for the integration of the Ri-plasmid DNA (which can be integrated into plant genome in two fragments: T left ($T_L$), and T right ($T_R$); White et al., 1985), two plants did not contain any Ri-plasmid T-DNA, one plant had a 5.7 kb fragment of the $T_R$-DNA, and two plants had different amounts of the $T_L$-DNA. This demonstrates that the method of this invention does not select strongly for or against $T_L$-DNA or $T_R$-DNA. Table II below summarizes the analysis proving the integration of the vector DNA as well as Ri plasmid DNA (both fragments), as follows:

7

## TABLE II

Integration of the vector DNA (NPT) and the Ri-plasmid DNA ($T_L$ and $T_R$) into DNA of five cucumber plants as a result of transformation with <u>A. rhizogenes</u>.

| | | Hind III fragments(kb)[1] | | |
|---|---|---|---|---|
| Plant # | <u>A. Rhizogenes</u> Vector Plasmid | Vector T-DNA (NPT) | $T_L$-DNA | $T_R$-DNA |
| 1 | pARC8 | 5.7; 4.9 | 0 | 5.7 |
| 2 | pACR8 | 12.0; 4.8 | 0 | 0 |
| 3 | pARC16 | 9.4; 4.9 | 0 | 0 |
| 4 | pARC16 | 4/8; 3.9 | 3.4  1 5.9 | 0 |
| 5 | pARC16 | 6.4; 3.7 | 3.4 | |

1) The probes were pNEO 105 (vector DNA; Simpson et al., 1986), pFW94 or pFW41 ($T_L$-DNA and $T_R$-DNA, respectively; Huffman et al., 1984)

2) This agrees with the mobility expected for the internal Hind III fragment (H-21) of the $T_L$-DNA, as described by White et al. (1985).

The tranfer of the vector-DNA only, vector plus Ri-DNA, or Ri-DNA only illustrates the flexibility of this system of transformation. The transfer of the vector-DNA only as observed in plants 2 and 3 (Table II) shows that a transfer of a desired gene can be achieved in cucumber using the A. rhizogenes strain without removing the Ri-DNA from the resident plasmid. In other words the resident Ri-plasmid does not have to be disarmed. On the other hand, since some of the characteristics associated with the Ri-plasmid DNA (like shorter internodes and male sterility) may also be desirable, Table II shows that cucumber plants having both the vector and the Ri-DNA can be created and selected. The method of transformation of this invention also permits recovery of plants containing only the Ri-DNA, which plants thus will be compact and male sterile, if such characteristics only are desired.

Figure 4 shows a normal, control plant of C. sativus L., c.v. Straight Eight on the left, as compared to a transgenic plant of this invention, "PCRI HiDensity", on the right. Generally, the PCRI HiDensity plants may be described as trailing, annual herbs with branched hirsute vines and tendrils. Leaves are alternate, simple with petioles, palmately 3- to 5-lobed or angular, and thicker and narrower, pointed leaves may be expected in some plants. Tendrils are simple, lateral, stipular in position, one at each node. Transformed plants may be characterized from the control or wild plants in having shorter than normal internodes, on the order of 2.5 - 7.5 cm as compared to the normal 10 - 15 cm, resulting in a compact morphology. The flowers are monoecious, but gynoecious, perfect, andromonecious and trimonecious forms are also expected to occur. The flowers are yellow or cream colored, fascicled or solitary, and often borne at every node. The staminate flowers have the calyx and corolla united to form a tubular receptacle, stamens basically 5, alternate with petals, filaments free or united, anthers free or coherent in a head. The pistillate flowers have the calyx and corolla similar to the male flowers, ovary inferior usually with one locule and 3 or 4 carpels, style solitary with 2- or 3-lobed stigma. The fruit is a fleshy berry, indehiscent. The seeds are large, numerous, white or tan colored, with no endosperm, and embryos have large cotyledons. The seeds from the fruit of compact (short internodal) genetically transformed plants of this invention are germinable and have been grown into the R2 generation to date.

Upon maturation, the transgenic as well as the regenerated control cucumbers displayed varing degrees of diminution and abscission of male flowers. The abscission of male flowers in the control plants (which were regenerated but not transformed indicates that the reduced fertility most likely did not result from transformation, as was reported in other species (Tepfer, 1984), but rather was due to abnormalities associated with the regeneration procedures. In heritable form, these genetic alterations are significant and beneficial in Cucumis (particularly C. sativus) breeding, since compact stature and male sterility are desirable in this species (Kauffman and Lower, 1976). Thus, shortened internodes developed in these plants permit production of the same fruit on lesser acreage, at correspondingly lower costs. Stated conversely, more fruit can be produced from the same acreage.

Surprisingly, none of the transgenic plants showed abnormalities in leaf morphology, such as leaf wrinkling as expected from the reports of tobacco, carrot and morning glory transformed with sA. rhizogenes (Tepfer, 1984).

Using the methods of this invention, we recovered transgenic cucumber plants within 10 weeks. This methodology is simpler and faster than the co-cultivation method (Marton et al., 1979) in which plant protoplasts are transformed. The co-cultivation method is labor-intensive and prone to contamination, whereas the techniques of this invention consist of a few, simple steps that can be effectively performed in standard laboratories. Furthermore, somaclonal variation frequently results from protoplast culture, whereas organized tissue is known to remain more stable (Shepard et al., 1980; Krens et al., 1982; Horsch et al., 1985).

Although our work indicates that some cucumber cultivars do not regenerate plants from roots under the specified conditions (Trulson and Shahin, 1986; or copending case 92/9), the cultivars discovered by us which are capable of root regeneration (Sunblest, Burpless, Bush Slicer, GY 14, Straight 8) can serve as intermediates in gene transfer to other genetic backgrounds. Additionally, since the genotypes capable of regeneration from roots represent highly advanced germplasm, genetic engineering performed in this germplasm will help to advance already superior genotypes.

The methods of transformation of this invention also facilitate somatic hybridization (including both hybrids and cybrids) in Cucumis sp. plants, particularly cucumber. Although the potential of somatic hybridization is well recognized, the previous lack of selectable markers constitutes a major obstacle in manipulating protoplasts of higher plants (Cocking et al., 1981. Thus, the methods of this invention permit the introduction of drug-resistance markers to aid in identification and selection of desirable protoplast (or $p_x/c_y$) fusion products.

Further, DNA encoding chloramphenicol-resistance can be transferred to chloroplasts (Van den Brock et al., 1985). Thus, with the methods and markers of this invention, kanamycin and chloramphenicol resistance can be introduced into plant tissue by appropriate vectors, and chloramphenical and kanamycin can be used to select for nucleus/chloroplast fusion products with the desired marked traits. Thus, somatic hybrid isolation and selection is facilitated. Moreover, these markers would be of great value if transfers of agronomically important genes, since the presence of an easily identifiable marker linked to an agronomically desirable gene would permit efficient selection in tissue culture (Fraley et al., 1983; Herrera-Estrella et al., 1983).

### Example B. Transformation/Somatic Hybridization.

Somatic hybridization is accomplished by protoplast-protoplast or protoplast-cytoplast fusion (generically $p_x/c_y$ fusion). The protoplasts and/or cytoplasts of different species, cultivars or genetically altered plants (such as the transgenic plants of this invention) having desired traits should be separately liberated, prepared, fused and regenerated as described in our copending case 92/13.

Transformation may precede, or follow, somatic hybridization, or may be sequentially combined with electroporation, cocultivation or chemical fusion techniques, and somaclonal variation selection via culturing, to assist in isolating and selecting plants with desired traits.

Further, these fusion techniques can also be used to genetically transform protoplasts by fusing them with liposomes (phospholipid bilayer vesicles) that may contain foreign DNA, or bacterial sphereoplasts, which are bacterial cells devoid of cell walls.

### Example C. Encapsulation of Transgenic Embryoids -

Artifical Seeds. Since in vitro production of somatic embryoids as in Example A5 above results in embryoids without protective seed coats, the transgenic embryoids of this invention may be encapsulated, individually or in groups, in capsules or coatings to retard dehydration and preserve them for future "planting" and growing as set forth in our copending cases 92/9 or 92/13.

### Example D. Regeneration After Freeze Storage of Transgenic

Embryoids/Mini-Calli. One or more of the transgenic protoplastt sources, protoplasts therefrom, mini-calli, or embryoids may be stored frozen for extended periods, then thawed and regenerated following the steps and media set forth in our copending cases 92/9 and 92/13.

It should be understood that various modifications within the scope of this invention can be made by one of ordinary skill in the art without departing from the spirit thereof. For example, the positive response of Cucumis sp. plants to transformation and regeneration by the media and methods of this invention render them useful in breeding. One important application is in the production of gynoecious populations to replace the current expensive treatment with silver nitrate. Another application of this invention is use of the techniques herein in combination with somatic hybridization and/or somaclonal variation as a valuable source of diversity in plant material used in breeding. The fusion of transformed protoplasts or transformation of $p_x/c_y$ fusion, followed by regeneration may be used for selection of individuals resistant to pathogens, toxic metals, pesticides and herbicides. The latter is of particular importance because cucumbers are very sensitive to herbicides.

The application of genetic engineering in cucumber and muskmelon breeding is of special value. These two species are sexually incompatible. Conventional crosses and transfer of many desirable traits like disease resistance heretofore have not been possible (Deakin et al., 1971). Such barriers are now removed with the development of capability of gene transfer through plant transformation disclosed herein, used alone or in combination with somatic hybridization disclosed in our copending case 92/13. Further, the explant regeneration culture techniques of our copending case 92/9 leads to selection of desirable traits through somaclonal variation.

In addition to the ability to transfer horticulturally desirable genes into cucumber or muskmelon, genetic engineering procedures are particularly valuable in introduction of marker genes such as resistance to kanamycin or chloramphenicol. These markers facilitate somatic hybridization via protoplast fusion, thus removing the sterility barriers in the genus Cucumis.

We therefore wish our invention to be defined by the scope of the appended claims as broadly as the prior art will permit, and in view of this specification if need be.

References

1. Bevan, M. (1984): Binary Agrobacterium vectors for plant transformation. Nucleic Acid Res 12:8711-8121.

2. Chilton, M-D., Currier, T.C., Farrand, S.K., Bendich, A.J., Gordon, M.P., and Nester, E.W. (1974): Agrobacterium tumefaciens DNA and PS8 bacteriophage DNA not detected in crown gall tumors. Proc Nat Acad Sci USA 71:3672-3676.

3. Chilton, M-D., Tepfer, D.A., Petit, A., David, C., Casse-Delbart, F., and Tempe, J. (1982): Agrobacterium rhizogenes inserts T-DNA into the genomes of the host plant root cells. Nature (London) 295:432-434.

4. Cocking, E.C., Davey, M.R., Pental, D., and Power, J.B. (1981): Aspects of plant genetic manipulation. Nature (London) 293:265-269.

5. David, C., Chilton, M-D., and Tempe, J. (1984): Conservation of T-DNA in plants regenerated from hairy root cultures. Biotechnology 2:73-76.

6. Deakin, J.R., Bohn, G.W., and Whitaker T.W. (1971) : Interspecific hybridization in Cucumis. Econ Bot 25:195-211.

7. Fraley, R.T., Rogers, S.G., Horsch, R.B., Sanders, P.R., Flick, J.S., Adams, S.P., Bittner, M.L., Brand, L.A., Fink, C.L., Fry, J.S., Galluppi, G.R., Goldberg, S.B., Hoffmann, N.L., and Woo, S.C. (1983): Expression of bacterial genes in plant cells. Proc Natl Acad Sci USA 80:4803-4807.

8. Herrera-Estrella, L., Depicker, A., VanMontagu, M., and Schell, J. (1983) : Expression of chimaeric genes transferred into plant cells using a Ti-plasmid-derived vector. Nature (London) 303:209.

9. Hoekema, A., Hirsch, P.R., Hooykaas, P.J.J., and Schilperoort, R.A. (1983) : A binary plant vector strategy based on separation of vir- and T-region of the Agrobacterium tumefaciens Ti-plasmid. Nature (London) 303:179-180.

10. Horsch, R.B., Fry, J.E., Hoffmann, N.L., Eichholtz, D., Rogers, S.G., and Fraley, R.T. (1985) : A simple and general method of transferring genes into plants. Science 227:1229-1231.

11. Huffman, G.A., White, F.F., Gordon, M.P., and Nester, E.W. (1984) : Hairy-root-inducing plasmid: physical map and homology to tumor-inducing plasmids. J. Bacteriol 157:269-276.

12. Jones, J.D.G., Dunsmuir, P., and Bedbrook, J. (1985) : High level expression of introduced chimaeric genes in regenerated trnsformed plants. (EMBO J 4:2411-2418).

13. Kauffman, C.S., and Lower, R.L. (1976) : Inheritance of an extreme dwarf plant type in the cucumber. J. Am Soc Hort Sci 101:150-151.

14. Krens, F.A., Molendijk, L., Wullems, G.J., and Schilproort, R.A. (1982) : In vitro transformation of plant protoplasts with Ti-plasmid DNA. Nature (London) 290:72-74.

15. Larkin, P.J., and Scowcroft, W.R. (1981) : Somaclonal variation - a novel source of variability from cell cultures for plant improvement. Theor Appl Genet 60:197-214.

16. Marton, L., Wullems, G.J., Molendijk, L., and Schilperoort, R.A. (1979) : In vitro transformation of cultured cells from Nicotiana tabacum by Agrobacterium tumefaciens. Nature (London) 277:129-131.

17. Murashige, T., and Skoog, F., (1962) : A revised medium for rapid growth and bioassays with tobacco tissue cultures. Physiol Plant 15:473-497.

18. Reiss, B., Sprengel, R., Will, H., and Schaller, H., (1984) : A new sensitive method for qualitative and quantitative assay of neomycin phosphotransferase in crude cell extracts. Gene 30:211:218.

19. Saghai-Maroof, M.A., Soliman, K.M., Jorgensen, R.A., and Allard, R.W. (1984) : Ribosomal DNA spacer-length polymorphisms in barley: Mendelian inheritance, chromosomal location, and population dynamics. Proc Natl Acad Sci USA 81:8014-8018.

20. Shahin, E.A. (1985) : Totipotency of tomato protoplasts. Theor Appl Genet 69:235-240.

21. Shepard, J.F., Bidney, D., and Shahin, E. (1980) : Potato protoplasts in crop improvement. Science 208:17-24.

22. Simpson, R.B., Spielmann, A., Margossian, L., and McKnight, T.D. (1986) : A disarmed binary vector from Agrobacterium tumefaciens functions in Agrobacterium rhizogenes: frequent co-transformation of two distinct T-DNA's. Plant Mol Biol. In Press.

23. Southern, E.M. (1975) : Detection of specific sequences among DNA fragments separated by gel electrophoresis. J Mol Biol 98:503-517.

24. Tepfer, D. (1984) : Transformation of several species of higher plants by Agrobacterium rhizogenes: sexual transmission of the transformed genotypoe and phenotype. Cell 37:959-967.

25. Thomashow, M.F., Nutter, R., Montoya, A.L., Gordon, M.P., and Nester, E.W. (1980) : Integration and organization of Ti plasmid sequences in crown gall tumors. Cell 19:729-739.

26. Trulson, A.J., and Shahin, E.A. (1986) : In vitro plant regeneration in the genus Cucumis. In preparation.

27. Van den Broeck, G., Timko, M.P., Kausch, A.P., Cashmore, A.R., Van Montagu, M., and Herrera-Estrella, L. (1985) : Targeting of a foreign protein to chloroplasts by fusion to the transit peptide from the small subunit of ribulose 1,5-biphosphate carboxylase, Nature (London) 313:358-363.

28. White, F.F., Taylor, B.H., Huffman, G.A., Gordon, M.P., and Nester, E.W. (1985) : Molecular and genetic analysis of the transferred DNA regions of the root-inducing plasmid of Agrobacterium rhizogenes. J Bacteriol 164:33-44.

29. Zambryski, P., Joos, H., Genetello, C., Leemans, J., VanMontagu, M., and Schell, J. (1983) : Ti plasmid vector for the introduction of DNA into plant cells without alteration of their normal regeneration capacity. EMBO J 2:2143-2150.

## Claims

1. The new and distinct variety of cucumber plant, PCRI Hi-Density, shown and described, and the seeds and embryoids or mini-calli thereof.

2. A method of transforming plant tissue which comprises
inoculating said plant tissue with A. rhizogenes containing a vector in addition to the resident Ri plasmid for a time sufficient to introduce said vector to at least some of the cells of said tissue to form at least one transgenic cell, and
optionally culturing at least one of said transgenic cell(s) in an appropriate medium for a time sufficient to regenerate a competent plant structure selected from a competent colony of cells, tissue, mini-calli, embryoid, plantlet, plant and seed.

3. The method of claim 2 wherein said plant tissue is hypocotyl tissue.

4. The method of claim 2 wherein said plant tissue is from a member of Cucumis sp.

5. The method of claim 1 wherein said culturing step includes culturing on CTM-3 Medium, followed by culturing on CTM-3 to form a mature transgenic embryoid, and transferring said embryoid to CTM-4 Medium for development into a plantlet.

6. Competent transgenic mini-calli or embryoids produced by the process of claim 2.

7. A method of culturing competent transgenic mini-calli or embryoids produced by the method of claim 2 and frozen which comprises
thawing said frozen transgenic embryoid or mini-calli; and
culturing said thawed transgenic embryoid or mini-calli to promote further development and maturation

8. A method of obtaining a transgenic plant tissue which comprises the steps of:
    a) fusing one ore more cellular-derived material(s) selected from one or more protoplast(s) , cytoplast(s) , sphereoplast(s) , liposome(s) and mixtures thereof, from one recipient plant, with one or more cellular-derived material selected from one ore more protoplast(s) , sphereoplast(s) , liposome(s) , cytoplasts and mixtures thereof from another, donor organism to produce a fusion product; and
    b) culturing said fusion product for a time sufficient to produce a viable genetically altered mini-calli or embryoid.

9. The method of claim 8 wherein said fusion is selected from $p_x/c_y$ fusion, and said donor material source is selected from plant and bacterial cell material.

10. The method of claim 9 wherein said material source for both said donor and recipient is from Cucumis sp. plant cells.

0262972

26·01·88

Fig. 1.

Fig. 3.

1 2 3 4 5 6 7 8 9 10

-NPT

Fig. 2.

Fig. 4.

CONTROL    TRANSGENIC